(19) Europäisches Patentamt
European Patent Office
Office européen des brevets

(11) Veröffentlichungsnummer : **0 166 283**
**B1**

(12) **EUROPÄISCHE PATENTSCHRIFT**

(45) Veröffentlichungstag der Patentschrift : **25.05.88**

(21) Anmeldenummer : **85107053.2**

(22) Anmeldetag : **07.06.85**

(51) Int. Cl.⁴ : **C 07 C 51/353**, C 07 C 57/42, C 07 C 57/44, C 07 C 57/60, C 07 C 59/68

(54) **Verfahren zur Herstellung von gegebenenfalls kernsubstituierten Zimtsäuren.**

(30) Priorität : **20.06.84 DE 3422915**

(43) Veröffentlichungstag der Anmeldung : **02.01.86 Patentblatt 86/01**

(45) Bekanntmachung des Hinweises auf die Patenterteilung : **25.05.88 Patentblatt 88/21**

(84) Benannte Vertragsstaaten : **CH DE FR GB IT LI NL**

(56) Entgegenhaltungen :
**THE JOURNAL OF ORGANIC CHEMISTRY, Band 15, Nr. 3, Mai 1950, Seiten 451-456, Baltimore, US; J.F.J. DIPPY u.a.: "The nature of catalyst in the perkin condensation"**

(73) Patentinhaber : **BAYER AG**
**Konzernverwaltung RP Patentabteilung**
**D-5090 Leverkusen 1 Bayerwerk (DE)**

(72) Erfinder : **Beitzke, Bernhard, Dr.**
**Bergmannsweg 5**
**D-5060 Bergisch Gladbach 1 (DE)**
Erfinder : **Handschuh, Volkmar, Dr.**
**Luisenstrasse 19**
**D-5093 Burscheid (DE)**
Erfinder : **Blank, Heinz Ulrich, Dr.**
**Am Geusfelde 35**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 166 283 B1

**Beschreibung**

Die vorliegende Erfindung betrifft ein verbessertes Verfahren zur Herstellung von gegebenenfalls kernsubstituierten Zimtsäuren durch Perkin-Reaktion aus einem gegebenenfalls kernsubstituierten aromatischen Aldehyd, Acetanhydrid und alkalischen Kondensationsmitteln.

Die Perkin-Reaktion ist an sich bekannt (Houben-Weyl, 4. Auflage, Band VIII, Seite 442 ff. (1952) ; Org. React. I, Seite 210-265 (1942) ; Ullmanns Enzyklopädie der technischen Chemie, 3. Auflage, Band 19, Seite 36 (1969)). Hierbei werden alle Reaktionspartner zusammengegeben und gemeinsam längere Zeit unter Rückfluß erhitzt, wobei gegebenenfalls die entstehende Essigsäure abdestilliert wird. Als Kondensationsmittel nennt die DE-OS 15 68 184 Natrium-/Kalium-Acetat im Gemisch mit Kaliumcarbonat/-hydrogencarbonat und eine Reaktionstemperatur von 130-200 °C.

Bei dieser Reaktion entstehen durch Nebenreaktionen dunkelbraune Harze, die eine Extraktion der Cinnamat-Lösung mit einem organischen Lösungsmittel und/oder eine Klärung mit A-Kohle notwendig machen. Dennoch wird nach diesen Reinigungsoperationen, will man sie nicht mehrmals durchführen, eine gelb oder bräunlich verfärbte Zimtsäure erhalten. Der Aufwand für die Herstellung einer farblosen Zimtsäure ist also erheblich.

Zimtsäure ist ein Vorprodukt für die Herstellung von L-Phenylalanin, das aus der Säure durch enzymatische Aminierung hergestellt wird (Chem. Abstr. 89, 213 580 p. (1978), Chem. Abstr. 95, 95 470b (1981)). Für diese Biotransformation ist eine besonders reine Zimtsäure erforderlich, die frei von verfärbenden Verunreinigungen sein muß.

Als Verbesserung des Herstellungsverfahrens wird in der DE-OS 31 39 994 ein Nachdosieren von Kaliumcarbonat während der Reaktion beschrieben.

Nach den Angaben in der DE-OS 31 44 261 wird bei der Perkin-Reaktion ein reineres Endprodukt erhalten, wenn man das Acetanhydrid durch Erhitzen mit dem Kondensationsmittel zuerst enolisiert und dann während der Reaktionsdauer den Aldehyd gleichmäßig zulaufen läßt.

Überraschenderweise wurde jedoch gefunden, daß sich die Nebenreaktionen und die Harzbildung in beträchtlichem Umfange zurückdrängen lassen, wenn man entgegen der Lehre der DE-OS 31 44 261 das Acetanhydrid zu dem vorgelegten Benzaldehyd dosiert.

Es wurde nunmehr ein Verfahren zur Herstellung von gegebenenfalls kernsubstituierten Zimtsäuren durch Perkin-Reaktion aus gegebenenfalls kernsubstituierten Benzaldehyden, Acetanhydrid und Alkalisalzen von Carbonsäuren und/oder Alkali(hydrogen)carbonaten als Kondensationsmitteln gefunden, das dadurch gekennzeichnet ist, daß man zunächst 0-75 Mol-% Acetanhydrid, bezogen auf den umzusetzenden gegebenenfalls kernsubstituierten Benzaldehyd mit diesem Benzaldehyd und den genannten Kondensationsmitteln vorlegt und bei 100-200 °C umsetzt und im Laufe der Reaktion das restliche Acetanhydrid zudosiert, bis ein Molverhältnis von 1-1,5 Mol Acetanhydrid/Mol des gegebenenfalls kernsubstituierten Benzaldehyds erreicht ist.

Als Ausgangsstoffe für das erfindungsgemäße Verfahren seien gegebenenfalls substituierte Benzaldehyde der Formel

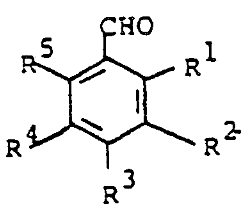

(I)

genannt, in der

$R^1$ Wasserstoff, Halogen, Alkyl oder Cycloalkyl bedeutet,

$R^2$, $R^3$ und $R^4$ unabhängig voneinander für Wasserstoff, Halogen, Alkyl, Cycloalkyl, Aryl oder Aryloxy stehen und

$R_5$ Wasserstoff, Halogen oder Methyl bedeutet,

und wobei weiterhin

bis zu vier beliebige Reste $R^1$ bis $R^5$ Alkoxy bedeuten können,

bis zu zwei beliebige Reste $R^1$ bis $R^5$ Nitro bedeuten können und

bis zu zwei beliebige Reste $R^2$ bis $R^4$ Cyano und/oder Alkoxycarbonyl bedeuten können.

In bevorzugter Weise werden aromatische Aldehyde der Formel

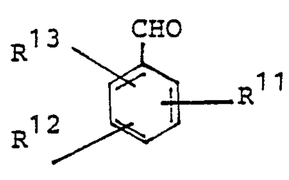

(II)

eingesetzt, in der $R^{11}$, $R^{12}$ und $R^{13}$ unabhängig voneinander Wasserstoff, Halogen, Alkyl, Aryl, Alkoxy oder Aryloxy bedeuten, wobei im Falle von Alkyl, das größer als Methyl ist, eine ortho-Position zur Aldehydgruppe Wasserstoff trägt und im Falle von Aryl beide ortho-Positionen zur Aldehydgruppe Wasserstoff tragen und in der weiterhin bis zu zwei der Reste $R^{11}$ bis $R^{13}$ unabhängig voneinander Trifluormethyl, Nitron, Cyano oder Alkoxycarbonyl bedeuten können, wobei im Falle von Cyano oder Alkoxycarbonyl beide ortho-Positionen zur Aldehydgruppe Wasserstoff tragen.

In besonders bevorzugter Weise werden aromatische Aldehyde der Formel

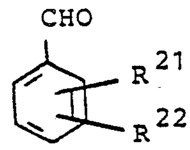

(III)

eingesetzt, in der $R^{21}$ und $R^{22}$ unabhängig voneinander Wasserstoff, Halogen, Aryl, Aryloxy, Alkoxy oder Nitro bedeuten, wobei im Falle von Aryl beide ortho-Positionen zur Aldehydgruppe Wasserstoff tragen.

In ganz besonders bevorzugter Weise werden aromatische Aldehyde der Formel

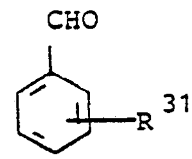

(IV)

eingesetzt, in der $R^{31}$ für Wasserstoff, Halogen steht.

Als Halogen sei beispielsweise Fluor, Chlor, Brom oder Jod, bevorzugt Fluor, Chlor oder Brom, besonders bevorzugt Fluor oder Chlor genannt.

Als Alkyl seien beispielsweise geradkettige oder verzweigte Kohlenwasserstoffreste mit 1-10 C-Atomen, bevorzugt mit 1-6 C-Atomen genannt. Alkyl schließt im Sinne des erfindungsgemäßen Verfahrens ein- oder mehrfach durch Halogen substituierte Kohlenwasserstoffreste ein, bevorzugt die Halogen-substituierten $C_1$-$C_6$-Kohlenwasserstoffreste, besonders bevorzugt Trifluormethyl. Als Cycloalkyl seien cyclische Kohlenwasserstoffreste mit 4-8 C-Atomen, bevorzugt 5-6 C-Atomen, genannt.

Als Aryl seien beispielsweise Phenyl, Naphthyl, Anthryl, Biphenylyl, bevorzugt Phenyl, genannt, die ein- oder mehrfach durch Methyl, Ethyl oder Halogen substituiert sein können.

In den Alkoxy-, Alkoxycarbonyl- bzw. Aryloxy-Substituenten haben die Alkyl- bzw. Arylgruppen den genannten Bedeutungsumfang.

Als erfindungsgemäß umzusetzende aromatische Aldehyde seien beispielhaft genannt : Benzaldehyd, Nitrobenzaldehyd (o, m, p), Methoxybenzaldehyd (o, m, p), 4-Phenylbenzaldehyd, Phenoxybenzaldehyd (o, m, p), Chlorbenzaldehyd (o, m, p), Fluorbenzaldehyd (o, m, p).

Als Kondensationsmittel werden eingesetzt : Alkalisalze von Carbonsäuren, beispielsweise Natriumformiat, Kaliumformiat, Natriumacetat, Kaliumacetat oder Alkali(hydrogen)carbonate wie beispielsweise Natriumcarbonat, Kaliumcarbonat, Rubidiumcarbonat oder Caesiumcarbonat bzw. die zugehörigen Hydrogencarbonate. Selbstverständlich können Gemische dieser Kondensationsmittel eingesetzt werden. Die Menge des Kondensationsmittels beträgt im allgemeinen 0,1-1,5 Äquivalent Alkalikation/Mol Benzaldehyd, bevorzugt 0,3-1,3 Äquivalente, besonders bevorzugt 0,5-1,1 Äquivalente. In bevorzugter Weise wird ein Gemisch von $Na^+$-Ionen mit Ionen der schweren Alkalimetalle eingesetzt, beispielsweise Natriumacetat/Kalium(hydrogen)carbonat oder Natriumacetat/Kaliumacetat, wobei das Kaliumsalz ganz oder teilweise durch Salze des Rubidiums und/oder caesiums ersetzt werden kann.

Das schwere Alkalimetall ist in einer solchen Mischung beispielsweise mit 5-50 Äquivalent-%, bevorzugt 10-40 Äquivalent-%, bezogen auf die Gesamtmenge an Alkalimetall-Äquivalenten vertreten.

Die Reaktion des erfindungsgemäßen Verfahrens wird so durchgeführt, daß man den gegebenenfalls substituierten Benzaldehyd und das Kondensationsmittel (gegebenenfalls ein Gemisch) in einem Reaktionsgefäß, gegebenenfalls in Gegenwart einer unterstöchiometrischen Menge an Acetanhydrid, vorlegt, auf die gewählte Reaktionstemperatur erhitzt und dann die restliche Menge des Acetanhydrids zudosiert und gegebenenfalls noch bis zum Ende der Umsetzung weiter erhitzt oder gegebenenfalls die Temperatur im Rahmen des genannten Bereiches steigert.

Die zusammen mit dem Benzaldehyd und dem (den) Kondensationsmittel(n) vorgelegte Menge an Acetanhydrid kann zwischen 0 und 75 Mol-%, bezogen auf den Aldehyd, liegen. Bevorzugt liegt die Menge an vorgelegtem Acetanhydrid bei 0-50, besonders bevorzugt bei 0-30 Mol-%. Gute Ergebnisse werden beispielsweise erhalten, wenn man 0,1-0,3 Mol Acetanhydrid/Mol Benzaldehyd vorlegt.

Die zuzudosierende Menge an Acetanhydrid liegt zwischen der vorgelegten Menge und einer Gesamtmenge von 1-1,5 Mol Acetanhydrid/Mol des gegebenenfalls substituierten Benzaldehyds. Die Gesamtmenge an Acetanhydrid beträgt in bevorzugter Weise 1,0-1,4 Mol, in besonders bevorzugter Weise 1,1-1,3 Mol pro Mol des gegebenenfalls substituierten Benzaldehyds.

Für den Fall der Verwendung von Alkali(hydrogen)carbonate wird ein Teil des Acetanhydrids, beispielsweise nach der Formel

$$K_2CO_3 + CH_3CO-O-COCH_3 \longrightarrow 2CH_3COOK + CO_2,$$

verbraucht. Dieser Acetanhydrid-Verbrauch ist in der genannten Gesamtmenge nicht enthalten und muß gegebenenfalls hinzugefügt werden.

Um die Harzbildung in erfindungsgemäßer Weise zurückzudrängen, genügt es, die Acetanhydrid-Dosierung nach Aufheizen des vorgelegten Gemisches im ersten Teil der Gesamtreaktionszeit vorzunehmen. Als erster Teil der Gesamtreaktionszeit sei beispielsweise das erste Zehntel oder das erste Achtel genannt; selbstverständlich kann man das Dosieren des Acetanhydrids aber auch während der gesamten Dauer der Reaktionszeit durchführen. Dabei ist es möglich, das Acetanhydrid zuerst mit einer höheren und dann im weiteren Verlauf der Reaktion mit einer geringeren Dosiergeschwindigkeit zuzugeben. Das zuzudosierende Acetanhydrid kann aber auch mit konstanter Geschwindigkeit bis zum Erreichen der gesamten vorgesehenen Menge an Acetanhydrid zudosiert werden. So wird beispielsweise bei einer Gesamtreaktionszeit von 8 Stunden ein deutlich helleres Reaktionsgemisch erhalten, wenn man erfindungsgemäß in der ersten Stunde das Acetanhydrid gleichmäßig zudosiert und dann noch 7 Stunden unter Rückfluß erhitzt, als wenn man gemäß dem Stand der Technik alle Reaktionspartner zusammen 8 Stunden unter Rückfluß erhitzt.

Bei der erfindungsgemäßen Verfahrensweise kann sowohl unter Rückfluß als auch unter Abdestillieren von Essigsäure gearbeitet werden. Will man die Reaktions-Essigsäure abdestillieren, so dosiert man das Acetanhydrid möglichst langsam zu ; hierbei wird die Acetanhydrid-Konzentration im Sumpf klein gehalten und die destillative Abtrennung der Essigsäure erleichtert.

Die erfindungsgemäße Reaktion wird in einem Temperaturbereich von 100-220 °C, bevorzugt 140-210 °C, besonders bevorzugt 150-190 °C, durchgeführt. Zweckmäßigerweise wird das vorgelegte Gemisch aus dem Benzaldehyd und Kondensationsmittel(n) und gegebenenfalls einem Teil des Acetanhydrids auf Rückflußtemperatur erhitzt und dann das restliche Acetanhydrid zudosiert. So kann man beispielsweise die Dosierung des Acetanhydrids bei einer Sumpftemperatur von 150-165 °C vornehmen.

In einer besonders bevorzugten Arbeitsweise legt man ein Gemisch aus dem gegebenenfalls substituierten Benzaldehyd und 10-30 mol-% Acetanhydrid (bez. auf den Aldehyd) zusammen mit dem (den) Kondensationsmittel(n) vor und setzt dieses Gemisch bei 100-150 °C um und dosiert dann zwischen 150 und 200 °C Sumpftemperatur das restliche Acetanhydrid im Laufe der Reaktion zu, bis ein Molverhältnis von 1 bis 1,5 mol Acetanhydrid pro mol (subst.) Benzaldehyd erreicht ist. Danach erhitzt man noch solange, bis kein Umsatz mehr nachzuweisen ist. Dies läßt sich mit chromatographischen Methoden (Gaschromatographie oder Hochdruckflüssigkeitschromatographie) feststellen.

Ein Vorteil der erfindungsgemäßen Arbeitsweise liegt darin, daß mit einer hohen Sumpftemperatur begonnen werden kann, die über dem Siedepunkt des Acetanhydrids liegt, wodurch eine hohe Reaktionsgeschwindigkeit erreicht wird. Dadurch können in kurzer Zeit hohe Ausbeuten erzielt werden.

Die Reaktion kann sowohl unter Normaldruck als auch unter erhöhtem Druck durchgeführt werden. Unter erhöhtem Druck arbeitet man vorteilhafterweise im oberen Teil des angegebenen Temperaturbereiches, wobei die Dosierung des Acetanhydrids beispielsweise durch Zupumpen erfolgt.

Die Reaktion sollte unter einem Schutzgas, beispielsweise Stickstoff, durchgeführt werden.

Es muß als ausgesprochen überraschend bezeichnet werden, daß durch die erfindungsgemäße Verfahrensweise der Acetanhydrid-Dosierung die Nebenprodukt- und Harzbildung weitgehend vermieden werden kann, während in der DE-OS 31 44 261 zur Erreichung des gleichen Zieles ausdrücklich die umgekehrte Arbeitsweise beschrieben wird. Insbesondere überraschend ist es, daß es genügt, während eines ersten Teiles der Gesamtreaktionszeit das Acetanhydrid zu dosieren, um die deutlichen Effekte der Zurückdrängung von Nebenprodukt- und Harzbildung (d.h. einer wesentlichen Farbverbesserung) zu erzielen.

Das erfindungsgemäße Verfahren ermöglicht

a) eine weitgehende Zurückdrängung der Harzbildung,

b) damit verbunden eine vereinfachte kürzere und billigere Aufarbeitung,

c) eine leichtere destillative Abtrennung der Reaktions-Essigsäure und

d) kürzere Reaktionszeiten durch höhere Sumpftemperaturen vom Anbeginn der Reaktion.

## Beispiel 1

In einem 2-l-Kolben mit Rührer, Rückflußkühler, Thermometer und Tropftrichter wurden 106,1 g Benzaldehyd, 61 g Natriumacetat und 18 g Kaliumcarbonat vorgelegt und auf 160 °C erhitzt. Dann wurden innerhalb von 80 Minuten 136 g Acetanhydrid gleichmäßig zugetropft. Danach erhitzte man noch 6 Stunden und 40 Minuten unter Rückfluß. Eine Probe von 27,1 g des so erhaltenen Reaktionsgemisches zeigte als 10 Gew.-%ige Lösung in Eisessig eine Farbzahl von 350 Hazen (APHA). Dann wurden die leicht flüchtigen Bestandteile abdestilliert (111 g). Der Rückstand wurde mit 700 ml Wasser und 250 ml Toluol versetzt. Die wäßrige Phase wurde auf pH 8 gestellt, worauf die Phasen getrennt wurden. Man extrahierte

noch zweimal mit je 200 ml Toluol und isolierte dann die Zimtsäure aus der wäßrigen Phase durch Ansäuern. Es wurden 96,6 g Zimtsäure erhalten mit einer Farbzahl von 200 Hazen (APHA), gemessen als 10 gew.-%ige Lösung in Eisessig. Die Zimtsäure hatte einen Gehalt von mehr 99 %.

Beispiel 2

Man verfuhr wie im Beispiel 1, nur wurde nach dem Zutropfen des Acetanhydrids nur 5 Stunden 40 Minuten unter Rückfluß erhitzt. Danach war die Reaktionsmischung hellgelb. Nach Aufarbeitung wie in Beispiel 1 wurden 104 g Zimtsäure, Fp 135-136,5 °C erhalten ; das entsprach 70 % der theoretischen Ausbeute. Das Produkt hatte eine Farbzahl von 200 Hazen.

Beispiel 3 (Vergleichsbeispiel)

Es wurden die gleichen Mengen wie in Beispiel 1 eingesetzt, aber man gab alle Reaktionspartner zusammen, erhitzte und hielt 8 Stunden unter Rückfluß. Danach hatte eine Probe der Reaktionsmischung (wie in Beispiel 1 gemessen) eine Farbzahl von mehr als 500 Hazen (Farbzahl 30 auf der Jodskala bzw. Farbzahl 9 nach Gardner).

Beispiel 4

In einen 2-l-Vierhalskolben mit Rührer, Thermometer, Tropftrichter (500 ml) mit $N_2$-Aufleitung, Destillationsvorrichtung wurden 318,4 g Benzaldehyd, 182 g Natriumacetat und 47 g Kaliumcarbonat vorgelegt und auf 165 °C aufgeheizt. Bei konstanter Sumpftemperatur von 165 °C wurden innerhalb von 260 Min. 385 ml (408 g) Acetanhydrid gleichmäßig zugetropft. Nach Zugabe von ca. 150 ml Acetanhydrid stellte sich am Kolonnenkopf Rückfluß ein, und ein Destillat wurde bei Normaldruck und Kopftemperatur = 117 °C (maximal 118,5 °C) abgenommen. Nach dem Ende der Acetanhydrid-Zugabe wurde noch solange Destillat abgenommen, wie das bei 165° Sumpftemperatur möglich war (ca. 15 Min.). Eine Probe aus dem Reaktionsgemisch hatte eine Farbzahl von 350 Hazen (APHA) in der oben angegebenen Bestimmungsweise. nach Aufarbeitung analog Beispiel 1 wurden 318,1 g Zimtsäure, Gehalt 99,6 %, erhalten. Das entsprach einer Ausbeute von 71,3 % der theoretischen Ausbeute, bezogen auf den Einsatz an Benzaldehyd. Die Farbzahl des Produktes war 200 Hazen, gemessen als 10 %ige Lösung in Essigsäure.

Beispiel 5

In einer Apparatur, wie in Beispiel 1 beschrieben, wurden 318,4 g Benzaldehyd, 102 g Acetanhydrid, 182 g Natriumacetat und 54 g Kaliumcarbonat vorgelegt, auf 150 °C erhitzt und 30 min auf dieser Temperatur gehalten. Danach wurde auf 155-160 °C erhitzt und es wurden 306,4 g Acetanhydrid so zudosiert, daß eine Sumpftemperatur von 157 °C nicht unterschritten wurde. Die Gesamtreaktionszeit betrug 8 h.

Eine Probe aus dem Reaktionsgemisch hatte eine Farbzahl von 400 Hazen in der oben angegebenen Bestimmungsweise. Nach Aufarbeitung analog Beispiel 1 wurden 300 g Zimtsäure, Schmp. 135-136 °C, erhalten.

**Patentansprüche**

1. Verfahren zur Herstellung von gegebenenfalls kernsubstituierten Zimtsäuren durch Perkin-Reaktion aus gegebenenfalls kernsubstituierten Benzaldehyden, Acetanhydrid und Alkalisalzen von Carbonsäuren und/oder Alkali(hydrogen)carbonaten als Kondensationsmitteln, dadurch gekennzeichnet, daß man zunächst 0-75 Mol-% Acetanhydrid, bezogen auf den umzusetzenden gegebenenfalls kernsubstituierten Benzaldehyd, mit diesem Benzaldehyd und den genannten Kondensationsmitteln vorlegt und bei 100-220 °C umsetzt und im Laufe der Reaktion das restliche Acetanhydrid zudosiert, bis ein Molverhältnis von 1-1,5 Mol Acetanhydrid/Mol des gegebenenfalls substituierten Benzaldehyds erreicht ist.

2. Verfahren nach Anspruch 1, dadurch gekennzeichnet, daß gegebenenfalls kernsubstituierte Benzaldehyde der Formel

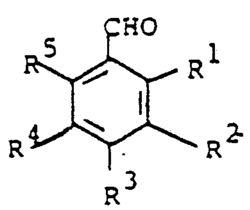

eingesetzt werden, in der

$R^1$ Wasserstoff, Halogen, Alkyl oder Cycloalkyl bedeutet,

$R^2$, $R^3$ und $R^4$ unabhängig voneinander für Wasserstoff, Halogen, Alkyl, Cycloalkyl, Aryl oder Aryloxy stehen und

$R^5$ Wasserstoff, Halogen oder Methyl bedeutet,

und wobei weiterhin
bis zu vier beliebige Reste $R^1$ bis $R^5$ Alkoxy bedeuten können,
bis zu zwei beliebige Reste $R^1$ bis $R^5$ Nitro bedeuten können und
bis zu zwei beliebige Reste $R^2$ bis $R^4$ Cyano und/oder Alkoxycarbonyl bedeuten können.

3. Verfahren nach Anspruch 1, dadurch gekennzeichnet, daß die Menge Acetanhydrid 0-75 Mol-%, bezogen auf den gegebenenfalls substituierten Benzaldehyd, beträgt.

4. Verfahren nach Anspruch 1, dadurch gekennzeichnet, daß die Menge Acetanhydrid 0-50 Mol-%, bezogen auf den gegebenenfalls substituierten Benzaldehyd, beträgt.

5. Verfahren nach Anspruch 1, dadurch gekennzeichnet, daß die Menge Acetanhydrid 0-30 Mol-%, bezogen auf den gegebenenfalls substituierten Benzaldehyd, beträgt.

6. Verfahren nach Anspruch 1, dadurch gekennzeichnet, daß die Menge Acetanhydrid 10-30 Mol-%, bezogen auf den gegebenenfalls substituierten Benzaldehyd, beträgt.

7. Verfahren nach Anspruch 1-6, dadurch gekennzeichnet, daß ein Molverhältnis von 1,0-1,4 Mol Acetanhydrid/Mol gegebenenfalls substituierten Benzaldehyd erreicht wird.

8. Verfahren nach Anspruch 1-6, dadurch gekennzeichnet, daß ein Molverhältnis von 1,1-1,3 Mol Acetanhydrid/Mol gegebenenfalls substituierten Benzaldehyd erreicht wird.

9. Verfahren nach Anspruch 1-8, dadurch gekennzeichnet, daß als Kondensationsmitel ein Salzgemisch mit $Na^+$-Ionen und Ionen der schweren Alkalimetalle eingesetzt wird, worin das oder die schweren Alkalimetalle mit 5-50 Äquivalent-% der Gesamtmenge an Alkalimetall-Äquivalenten vertreten ist (sind).

10. Verfahren zur Herstellung von gegebenenfalls kernsubstituierten Zimtsäuren durch Perkin-Reaktion aus gegebenenfalls kernsubstituierten Benzaldehyden, Acetanhydrid und Alkaliacetaten und/oder Alkalicarbonaten als Kondensationsmittel nach Anspruch 1-9, dadurch gekennzeichnet, daß man den Benzaldehyd mit 10-30 Mol-% (bezogen auf den Aldehyd) Acetanhydrid und den genannten Kondensationsmitteln vorlegt und bei 100-150 °C umsetzt, dan das restliche Acetanhydrid bei 150-200 °C im Laufe der Reaktion zudosiert, bis ein Molverhältnis von 1-1,5 Mol Acetanhydrid/Mol Benzaldehyd erreicht ist.

## Claims

1. Process for the preparation of cinnamic acids which are optionally substituted in the nucleus, by Perkin reaction, from benzaldehydes which are optionally substituted in the nucleus, acetic anhydride, and alkali metal salts of carboxylic acids and/or alkali metal (bi)carbonates as the condensing agents, characterized in that first 0-75 mol% of acetic anhydride, based on the benzaldehyde to be reacted, which is optionally substituted in the nucleus, is initially introduced together with the benzaldehyde and the condensing agents mentioned, and the reaction is carried out at 100-220 °C, and the remaining acetic anhydride is metered in during the course of the reaction until a molar ratio of 1-1.5 mole of acetic anhydride per mole of the optionally substituted benzaldehyde is reached.

2. Process according to Claim 1, characterized in that benzaldehydes which are optionally substituted in the nucleus, of the formula

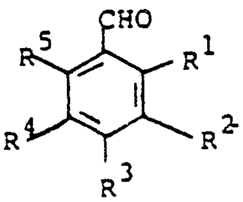

in which

$R^1$ denotes hydrogen, halogen, alkyl or cycloalkyl,

$R^2$, $R^3$ and $R^4$, independently of one another, represent hydrogen, halogen, alkyl, cycloalkyl, aryl or aryloxy and

$R^5$ denotes hydrogen, halogen or methyl,

wherein furthermore,
up to four of any radicals $R^1$ to $R^5$ can denote alkoxy,
up to two of any radicals $R^1$ to $R^5$ can denote nitro and
up to two of any radicals $R^2$ to $R^4$ can denote cyano and/or alkoxycarbonyl,
is used.

3. Process according to Claim 1, characterized in that the amount of acetic anhydride is 0-75 mol%, based on the optionally substituted benzaldehyde.

4. Process according to Claim 1, characterized in that the amount of acetic anhydride is 0-50 mol%, based on the optionally substituted benzaldehyde.

5. Process according to Claim 1, characterized in that the amount of acetic anhydride is 0-30 mol%, based on the optionally substituted benzaldehyde.

6. Process according to Claim 1, characterized in that the amount of acetic anhydride is 10-30 mol%, based on the optionally substituted benzaldehyde.

7. Process according to Claim 1-6, characterized in that a molar ratio of 1.0-1.4 mole of acetic anhydride per mole of optionally substituted benzaldehyde is reached.

8. Process according to Claim 1-6, characterized in that a molar ratio of 1.1-1.3 mole of acetic anhydride per mole of optionally substituted benzaldehyde is reached.

9. Process according to Claim 1-8, characterized in that the condensing agent used is a salt mixture containing $Na^+$ ions and ions of the heavy alkali metals, in which the heavy alkali metal(s) contribute(s) 5-50 equivalent% of the total amount of alkali metal equivalents.

10. Process for the preparation of cinnamic acids which are optionally substituted in the nucleus, by Perkin reaction, from benzaldehydes which are optionally substituted in the nucleus, acetic anhydride, and alkali metal acetates and/or alkali metal carbonates as the condensing agents according to Claim 1-9, characterized in that the benzaldehyde is initially introduced together with 10-30 mol% (based on the aldehyde) of acetic anhydride and the condensing agents mentioned, and the reaction is carried out at 100-150 °C, then the remaining acetic anhydride is metered in during the course of the reaction at 150-200 °C until a molar ration of 1-1.5 mol of acetic anhydride per mole of benzaldehyde is reached.

**Revendications**

1. Procédé de préparation d'acides cinnamiques éventuellement substitués dans le noyau par une réaction de Perkin à partir de benzaldéhydes éventuellement substitués dans le noyau, d'anhydride acétique et de sels alcalins d'acides carboxyliques et/ou de (bi)carbonates de métaux alcalins servant d'agents de condensation, caractérisé en ce que l'on mélange d'abord de 0 à 75 mol% d'anhydride acétique avec le benzaldéhyde éventuellement substitué dans le noyau mis en œuvre, par rapport à ce benzaldéhyde, et avec les agents de condensation en question, on fait réagir à des températures de 100 à 220 °C et on ajoute le reste d'anhydride acétique dans le cours de la réaction jusqu'à ce qu'on atteigne un rapport molaire de 1 à 1,5 mol d'anhydride acétique par mole du benzaldéhyde éventuellement substitué.

2. Procédé selon la revendication 1, caractérisé en ce que l'on part de benzaldéhydes éventuellement substitués dans le noyau répondant à la formule

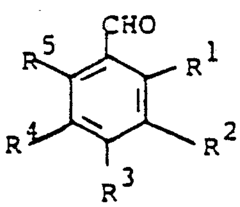

dans laquelle

$R^1$ représente l'hydrogène, un halogène, un groupe alkyle ou cycloalkyle,

$R^2$, $R^3$ et $R^4$ représentent chacun, indépendamment les uns des autres, l'hydrogène, un halogène, un groupe alkyle, cycloalkyle, aryle ou aryloxy, et

$R^5$ représente l'hydrogène, un halogène ou un groupe méthyle, avec les restrictions suivantes :

jusqu'à quatre substituants quelconques parmi les substituants $R^1$ à $R^5$ peuvent consister en groupes alcoxy,

jusqu'à deux substituants quelconques parmi les substituants $R^1$ à $R^5$ peuvent consister en groupes nitro, et

jusqu'à deux substituants quelconques parmi les substituants $R^2$ à $R^4$ peuvent consister en groupes cyano et/ou alcoxycarbonyle.

3. Procédé selon la revendication 1, caractérisé en ce que la quantité d'anhydride acétique est de 0 à 75 mol% par rapport au benzaldéhyde éventuellement substitué.

4. Procédé selon la revendication 1, caractérisé en ce que la quantité d'anhydride acétique est de 0 à 50 mol% par rapport au benzaldéhyde éventuellement substitué.

5. Procédé selon la revendication 1, caractérisé en ce que la quantité d'anhydride acétique est de 0 à 30 mol% par rapport au benzaldéhyde éventuellement substitué.

6. Procédé selon la revendication 1, caractérisé en ce que la quantité d'anhydride acétique est de 10 à 30 mol% par rapport au benzaldéhyde éventuellement substitué.

7. Procédé selon les revendications 1 à 6, caractérisé en ce que l'on atteint un rapport molaire de 1,0 à 1,4 mol d'anhydride acétique par mole de benzaldéhyde éventuellement substitué.

8. Procédé selon les revendications 1 à 6, caractérisé en ce que l'on atteint un rapport molaire de 1,1

à 1,3 mol d'anhydride acétique par mole de benzaldéhyde éventuellement substitué.

9. Procédé selon les revendications 1 à 8, caractérisé en ce que l'on utilise en tant qu'agent de condensation un mélange de sels contenant des ions $Na^+$ et des ions de métaux alcalins lourds, les ions de métaux alcalins lourds étant présents en quantité de 5 à 50 équivalents% par rapport à la quantité totale des équivalents de métaux alcalins.

10. Procédé de préparation d'acides cinnamiques éventuellement substitués dans le noyau par une réaction de Perkin à partir de benzaldéhydes éventuellement substitués dans le noyau, d'anhydride acétique et d'acétates alcalins et/ou de carbonates alcalins servant d'agents de condensation selon les revendications 1 à 9, caractérisé en ce que l'on mélange le benzaldéhyde avec 10 à 30 mol% (par rapport à l'aldéhyde) d'anhydride acétique et avec les agents de condensation mentionnés, on fait réagir à des températures de 100 à 150 °C et on introduit le reste d'anhydride acétique dans le cours de la réaction à des températures de 150 à 200 °C jusqu'à ce qu'on atteigne un rapport molaire de 1 à 1,5 mol d'anhydride acétique par mole de benzaldéhyde.